# FASCICULE DE BREVET EUROPEEN

(11) **EP 2 670 364 B1**
(45) Date de publication et mention de la délivrance du brevet: **28.01.2015**
(21) Numéro de dépôt: 12702300.0
(22) Date de dépôt: 03.01.2012
(51) Int. Cl.: A61F 5/02

(54) **CEINTURE DE SOUTIEN LOMBAIRE**
LUMBALER STÜTZGURT
LUMBAR SUPPORT BELT

(30) Priorité: 31.01.2011 FR 1150738
(43) Date de publication de la demande: 11.12.2013
(73) Titulaire: Gibaud, 42000 Saint Etienne (FR)
(72) Inventeur: ANGLADA, Gérard, 42000 Saint Etienne (FR); GIRARD, Frédéric, 75009 Paris (FR)
(74) Mandataire: Delorme, Nicolas
(86) Numéro de dépôt international: PCT/FR2012/050016
(87) Numéro de publication internationale: WO 2012/104510

(56) Documents cités:
- US-A1- 2005 054 960
- US-A1- 2009 082 707
- US-B1- 6 322 529

## Description

L'invention concerne une ceinture de soutien lombaire.

L'invention s'intéresse plus généralement au domaine de l'orthopédie dorsale.

Des pathologies fréquemment observées chez les patients sont des arthroses lombaires, des lombalgies, ou encore des lumbagos. Afin de soulager des patients sujets à ces pathologies, il est recommandé à ces patients de porter une ceinture de soutien lombaire. Une ceinture de soutien lombaire est une ceinture médicale réalisée dans une matière textile élastique, qui a pour fonction d'assurer chez le patient qui la porte un maintien de la région lombaire ainsi que de la région abdominale. Le port d'une ceinture lombaire a, notamment, pour but de réaliser un rappel de posture pour diminuer la douleur et mettre au repos la musculature contractée.

Une ceinture de soutien lombaire connue du déposant est par exemple décrite dans la demande de brevet US 6 322 529.

Un inconvénient inhérent de la ceinture lombaire décrite dans le document susmentionné est que la forme de la plaque dorsale ne permet pas un positionnement précis de la ceinture lombaire sur le patient. En particulier, il arrive fréquemment que lors de la mise en place de la ceinture, la plaque dorsale subisse une légère rotation selon un axe perpendiculaire au plan frontal du patient. La plaque dorsale est alors désaxée par rapport à l'axe médian de ce patient.

Un mauvais positionnement de la ceinture est préjudiciable dans la mesure où celui-ci peut conduire à une accentuation d'un défaut de posture du patient et peut ainsi entraîner l'aggravement de la pathologie dont souffre ce patient. L'invention vise ainsi à fournir une ceinture de soutien lombaire facilement positionnable.

Par ailleurs, afin qu'une ceinture de soutien lombaire produise un effet antalgique, il est nécessaire que la pression exercée par la ceinture sur la zone dorso lombaire du patient soit suffisamment élevée. A cet effet, le patient doit souvent produire un effort conséquent pour mettre en place la ceinture.

Or, généralement les patients souffrant d'arthrose lombaire sont également atteints d'arthrose des mains. Ces patients atteints d'arthrose des mains éprouvent de grandes difficultés à produire l'effort nécessaire pour mettre en place la ceinture convenablement.

Afin de pallier cet inconvénient, la ceinture décrite dans le document susmentionné comprend un mouflage pour démultiplier l'effort produit par le patient. Toutefois, l'effort demandé au patient reste conséquent.

L'invention vise à fournir une ceinture de soutien lombaire produisant une contention importante avec un minimum d'effort pour le patient.

L'invention a ainsi pour objet une ceinture de soutien lombaire, comportant :
- une plaque dorsale destinée à venir en appui contre la zone dorso lombaire d'un patient ;
- des première et seconde demi-ceintures destinées à venir en appui contre la zone lombo abdominale du patient, ces première et seconde demi-ceintures étant montées coulissantes avec la plaque ;
- un dispositif pour ajuster une pression exercée par la ceinture sur la zone dorso lombaire du patient, ce dispositif comprenant :
- des premières boucles fixées sur les extrémités proximales des première et seconde demi-ceintures ;
- des secondes boucles libres par rapport aux extrémités proximales des première et seconde demi-ceintures ;
- une sangle dorsale reçue à travers ces boucles en formant un maillage en ziz-zag.

De plus, selon une définition générale de l'invention :
- la plaque dorsale comporte des prolongements latéraux comprenant une arête inférieure conformée pour venir en appui contre les crêtes iliaques du patient, et
- le dispositif comprend également des première et seconde sangles de serrage fixées d'une part, sur une extrémité distale d'une demi ceinture respective, et reçues d'autre part à travers une seconde boucle libre respective, les première et seconde sangles de serrage étant destinées à être superposées aux prolongements latéraux de la plaque contre la zone lombo abdominale du patient.

La ceinture lombaire de l'invention est avantageuse en ce que les prolongements latéraux de la plaque dorsale permettent de positionner précisément la plaque dorsale sur le patient, et par la même permettent de positionner précisément la ceinture de soutien lombaire. En effet, lorsque le patient met en place la ceinture, celui-ci place les arêtes inférieures des prolongements latéraux directement en appui contre les crêtes iliaques. Le patient possède ainsi des repères fixes (à savoir les crêtes iliaques) permettant une excellente répétitivité du positionnement de la ceinture, et limitant le risque de mauvais positionnement de cette ceinture.

Du fait de la présence des prolongements latéraux, la plaque dorsale ne se résume pas à un dispositif d'appui dorsal, mais permet également un appui sur les zones latérales « molles » du patient, favorisant le serrage de la taille.

Les sangles de serrage facilitent l'actionnement du mouflage et permettent de démultiplier plus encore l'effort produit par le patient. Un avantage des sangles passant dans des boucles par rapport à des ficelles passant dans la gorge de poulies est que le risque de rupture ou de désengagement hors des boucles est considérablement réduit. En outre, l'épaisseur du système est réduite.

Enfin, une fois la pression ajustée, les sangles de serrage sont superposées aux prolongements latéraux de la plaque dorsale contre la zone lombo abdominale du patient. Une telle conformation des sangles de serrage provoque la déformation de la plaque dorsale qui prend la forme d'un « sablier ». Cette déformation produit une force de poussée entre les crêtes iliaques et la cage thoracique, dans une direction parallèle à l'axe médian du patient. Ainsi, la charge supportée par les vertèbres lombaire L5 et sacrée S1 est minimisée. La contention de la ceinture est ainsi améliorée.

Les modes de réalisation de cette invention peuvent comporter les caractéristiques suivantes.

La plaque dorsale peut comporter une ouverture centrale pour dégager les apophyses postérieures du patient, cette ouverture centrale s'étendant majoritairement selon un axe destiné à être positionné dans le plan sagittal du patient.

La plaque dorsale ainsi conformée est avantageuse en ce que la présence de l'ouverture centrale ne gêne pas les apophyses épineuses de la colonne du patient lorsque la plaque dorsale se déforme. Ainsi, la ceinture permet de s'adapter à la morphologie de chaque patient.

La plaque dorsale peut comprendre un rainage s'étendant selon un axe destiné à être positionné dans le plan sagittal du patient.

La plaque dorsale ainsi conformée est avantageuse en ce que le rainage guide la déformation de la plaque dorsale et minimise plus encore l'effort nécessaire pour déformer la plaque dorsale.

Il est également envisagé que les extrémités des parties libres des sangles de serrage forment une boucle pour recevoir un doigt du patient, de manière à faciliter l'exercice d'une force de traction sur les sangles de serrage par le patient.

Par ailleurs, les extrémités proximales des demi-ceintures peuvent comporter chacune au moins un passant à l'intérieur duquel est reçu un prolongement latéral de la plaque dorsale, de manière à permettre le coulissement des demi-ceintures avec la plaque dorsale.

Dans ces conditions, le montage et en alternance le démontage des demi-ceintures et la plaque dorsale est facilité.

Enfin, la ceinture peut comprendre des premier et second moyens de rappel élastiques raccordant la plaque dorsale d'une part et la, respectivement, première ou seconde demi-ceinture d'autre part, pour maintenir en permanence la plaque et les demi-ceintures centrées l'une avec l'autre. Des moyens sont cousus sur la plaque dorsale et sont fixés sur la demi-ceinture correspondante par des morceaux de tissu auto agrippant.

L'invention sera mieux comprise à la lecture de la description qui va suivre, donnée uniquement à titre d'exemple non limitatif et faite en se référant aux dessins sur lesquels :
La figure 1 est une illustration schématique d'une face intérieure d'une ceinture de soutien lombaire selon l'invention,
La figure 2 est illustration schématique d'une face extérieure de la ceinture de la figure 1
Les figures 3a et 3b sont des illustrations schématiques, en vue de dos, d'un patient portant la ceinture de soutien lombaire des figures 1 et 2, et
Les figures 3c et 3d sont des illustrations schématiques, respectivement en vue de face et en vue de côté du patient portant la ceinture de soutien lombaire des figures 1 et 2.

Ces figures sont orientées à l'aide d'un même référentiel orthogonal formé par des axes X, Y et Z perpendiculaires entre eux.

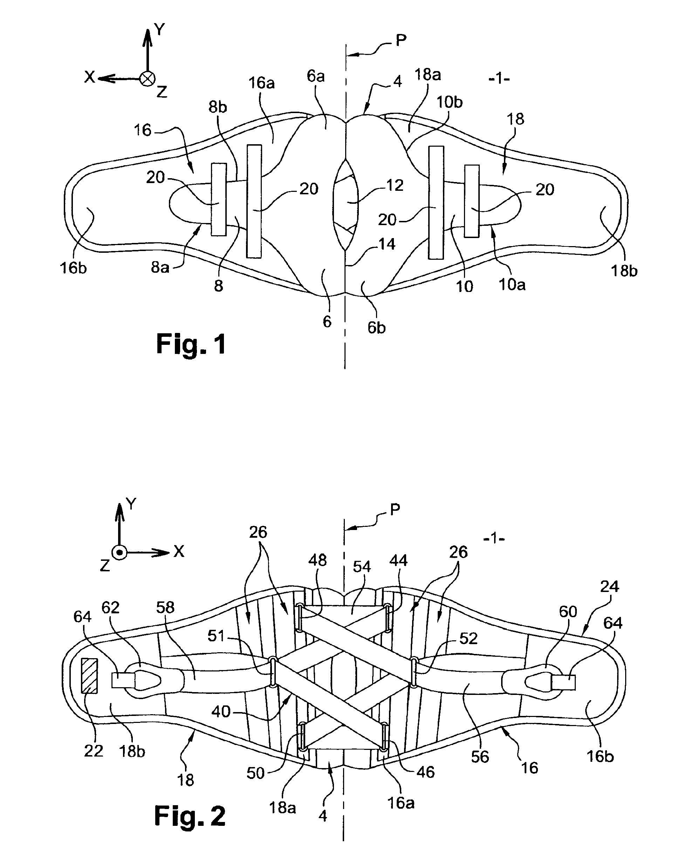

La figure 1 illustre une ceinture de soutien lombaire 1. La ceinture 1 comporte :
- une plaque dorsale 4 rigide,
- des demi-ceintures 16 et 18, et
- un dispositif 40 pour régler la contention de la ceinture 1 (plus visible sur la figure 2).

La plaque dorsale 4 peut être réalisée en mousse de polyuréthane de haute densité à structure ouverte. Cette matière permet de limiter l'effet de macération lors de l'utilisation de la ceinture. Cette mousse est capitonnée de tissu à bouclettes au contact du dos de l'utilisateur. La plaque dorsale peut également être réalisée en plastique thermoformable (à basse température d'application). Elle peut être modelée à la forme du dos de l'utilisateur.

La plaque dorsale 4 est formée d'un dossard 6 et de prolongements latéraux 8 et 10. La plaque dorsale 4 est de préférence formée d'une seule pièce.

Dans l'exemple illustré, le dossard 6 est de forme générale ovale. Le dossard 6 comprend une ouverture centrale 12 le traversant de part en part. Dans l'exemple, l'ouverture centrale 12 s'étend majoritairement selon un axe parallèle à l'axe Y. Ici, la section transversale de l'ouverture 12 est de forme ovale.

Le dossard 6 comprend un rainage 14. Dans l'exemple, le rainage 14 s'étend selon un axe parallèle à l'axe Y.

Les prolongements 8 et 10 s'étendent de part et d'autre du dossard 6 selon l'axe X. Les prolongements 8 et 10 ont une forme évasée, la largeur des prolongements 8 et 10, au niveau de leur extrémité distale respective étant plus faible que la largeur de ces prolongements 8 et 10 au niveau de leur jonction avec le dossard 6. La largeur des prolongements 8 et 10 est mesurée parallèlement à l'axe Y. Les prolongements 8 et 10 comprennent des arêtes inférieures, respectivement 8a, et 10a, et des arêtes supérieures respectivement 8b, et 10b.

Dans la suite de cette description, on appelle « plan de centrage de la plaque dorsale 4 » un plan formant un plan de symétrie pour cette plaque 4, ce plan étant parallèle aux axes Y et Z. Dans l'exemple, le plan de centrage est confondu avec un plan P. Ici, ce plan passe par l'axe selon lequel s'étend le rainage 14.

Les demi-ceintures 16 et 18 peuvent être réalisées dans un tissu élastique d'une force supérieure à 350 cN par centimètre à 30 % d'allongement. De préférence, le tissu est ajouré de manière à favoriser la ventilation.

Les demi-ceintures 16 et 18 comprennent respectivement :
- des extrémités proximales 16a et 18a, et
- des extrémités distales 16b, et 18b.

Les demi-ceintures 16 et 18 ont une forme évasée, la largeur des prolongements demi-ceintures 16 et 18, au niveau de leur extrémité distale 16b et 18b étant plus faible que la largeur de ces demi-ceintures 16 et 18 au niveau de leur extrémité proximale 16a et 18a. La largeur des demi-ceintures 16 et 18 est mesurée parallèlement à l'axe Y.

Les demi-ceintures 16 et 18 sont montées coulissantes avec la plaque dorsale 4. A cet effet, les extrémités proximales 16a et 18a comportent des passants 20 à l'intérieur desquels sont reçus les prolongements latéraux 8 et 10. Dans l'exemple, les extrémités proximales 16a et 18a comprennent chacune deux passants 20. Ici, ces passants 20 sont cousus sur la face intérieure des demi-ceintures 16 et 18.

La description fait désormais référence à la figure 2.

Les extrémités distales 16b et 18b comprennent des moyens de fermeture de la ceinture 1. Dans cet exemple, les moyens de fermeture sont formés par des rubans textiles 22 et 24, respectivement à boucles et à crochets. Ici, les rubans textiles 22 et 24 sont respectivement cousus sur la face intérieure de l'extrémité distale 16b, et sur la face extérieur de l'extrémité distale 18b.

Les demi-ceintures 16 et 18 comprennent de préférence des fourreaux 26 à l'intérieur desquels sont logées des baleines. Dans l'exemple, chaque demi-ceinture 16 et 18 comprend deux fourreaux 26 ménagés au niveau des extrémités proximales 16a et 18a. Les baleines sont par exemple en acier ressort.

Dans la suite de cette description, on appelle « plan de centrage des demi-ceintures 16 et 18 » un plan formant un plan de symétrie pour ces demi-ceintures 16 et 18, ce plan étant parallèle aux axes Y et Z. Ici, ce plan de centrage est confondu avec le plan P.

La ceinture 1 comporte de manière avantageuse des sangles de rappel élastiques (non illustrées) pour maintenir en permanence la plaque 4 et les demi-ceintures 16, 18 centrées l'une avec l'autre.

Par « maintenir centrées la plaque dorsale 4 et les demi-ceintures 16 et 18 l'une avec l'autre » on entend maintenir les plans de centrage de la plaque 4 et des demi-ceintures 16 et 18 confondus.

Les sangles de rappel sont fixées d'une part à la face extérieure du dossard 6 et d'autre part à la face intérieure d'une demi-ceinture 16,18 respective. A titre d'exemple, ici les sangles de rappel sont cousues au dossard 6 d'une part. D'autre part, les sangles de rappel comprennent un ruban textile à boucle pour se fixer à un ruban à crochets cousu sur la face intérieure de leur demi-ceinture 16, 18 respective.

Les demi-ceintures 16 et 18 sont mécaniquement raccordées l'une à l'autre par le biais du dispositif 40. Le dispositif 40 forme un mouflage comprenant :
- des boucles 44, 46 fixées sur l'extrémité proximale 16a,
- des boucles 48, 50 fixées sur l'extrémité proximale 18a,
- des boucles 51, 52 libres par rapport aux extrémités proximales 16a et 18a,
- une sangle dorsale 54 reçue à travers les boucles 44, 46, 48, 50, 51 et 52 en formant un maillage en ziz-zag, et
- des sangles de serrage 56, 58.

Les sangles de serrage 56, 58 comprennent une extrémité fixée, respectivement, aux extrémités distales 16b, 18b et une extrémité libre. Les sangles 56, 58 sont reçues à travers les boucles, respectivement 52 et 51.

De préférence, les sangles 54, 56, et 58 sont réalisées dans un tissu non extensible.

Dans l'exemple, les extrémités libres des sangles 56, 58 comprennent des passants respectivement 60, et 62 pour recevoir un doigt d'un patient. Aussi, le diamètre de ces passants 60, 62 est de préférence supérieur à 3 cm, 5 cm ou 10 cm. Les passants 60, 62 peuvent être pourvus de cavaliers textile qui protègent la commissure lors de la mise en traction et permettent une poussée confortable et intuitive.

Les extrémités libres des sangles 56, 58 comprennent des moyens 64 pour fixer, et en alternance libérer les sangles 56, 58 des demi-ceintures 16. Dans l'exemple, les moyens 64 sont des rubans de textiles à boucles pouvant se fixer à des rubans (non illustrés) à crochets cousus sur les faces extérieures des demi-ceintures 16, 18.

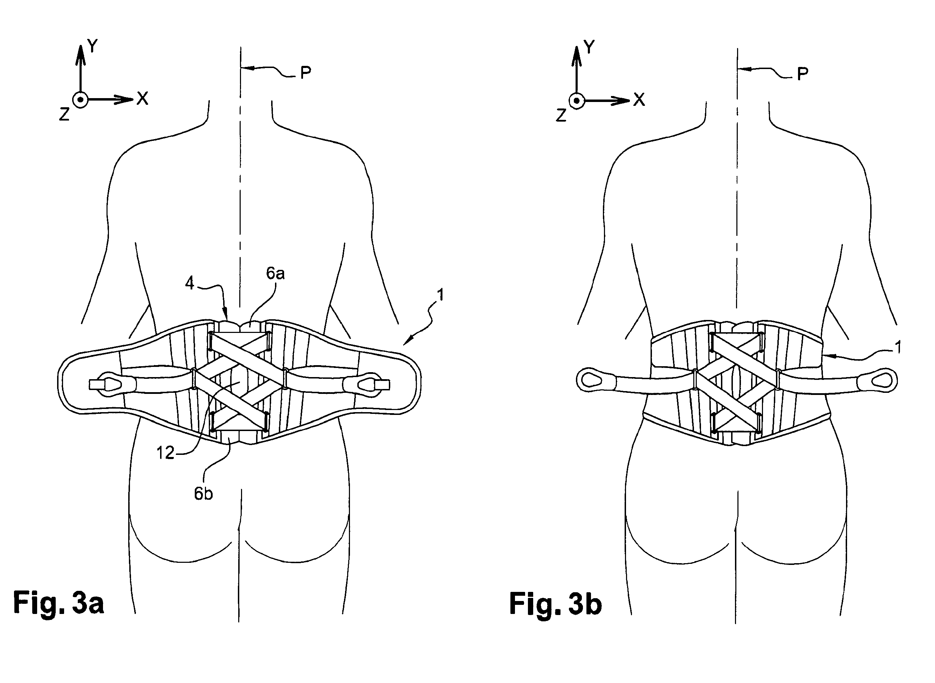

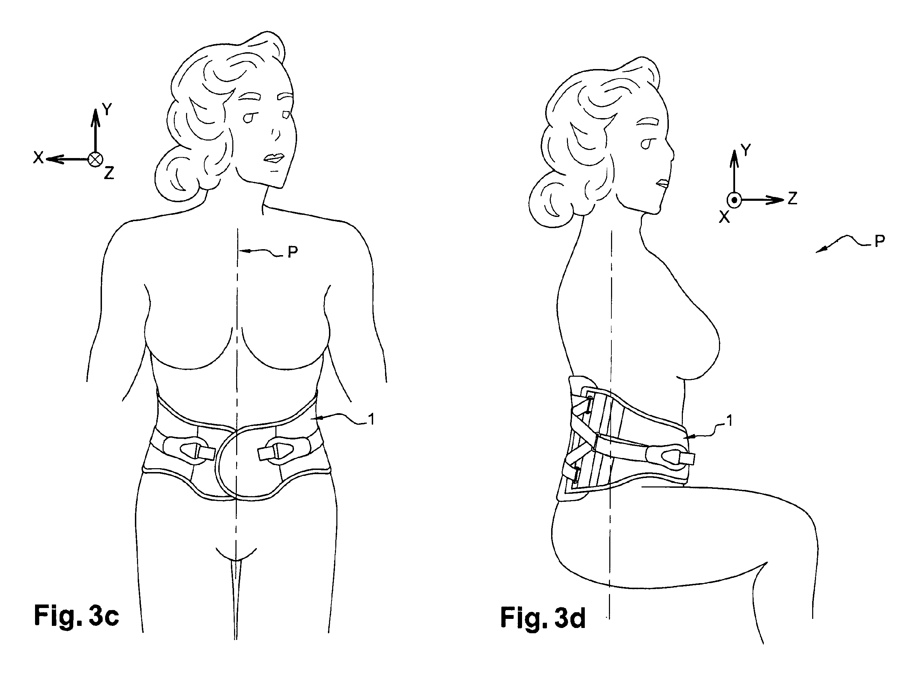

La mise en oeuvre de la ceinture 1 est maintenant décrite en référence aux figures 3a, 3b, 3c et 3d.

Dans la suite de cette description, on désigne par « axe médian » d'un patient l'axe passant par le sommet du crâne du patient et par le centre de gravité de ce patient. Dans l'exemple, l'axe médian est parallèle à l'axe Y.

On désigne par « plan sagittal » du patient un plan parallèle à l'axe médian et passant par un point situé à mi-distance entre les deux yeux du patient. Ici, le plan sagittal est parallèle aux axes Y et Z.

Lors d'une première étape illustrée sur la figure 3a, le patient positionne la ceinture 1. A cet effet, le patient saisit la ceinture 1 par le biais des extrémités distales 16b et 18b et positionne le dossard 6 de la plaque 4 en appui contre la zone dorso lombaire. Le dossard 6 est positionné de telle sorte que :
- une partie supérieure 6a du dossard 6 vienne en appui contre la vertèbre dorsale D11 du patient,
- une partie inférieure 6b vienne en appui contre la vertèbre sacrée S2 du patient, et
- l'ouverture 12 soit placée en vis-à-vis des apophyses postérieures du patient.

Dans le même temps, le patient dispose les prolongements latéraux 8 et 10 en appui contre la zone lombo abdominale de manière à ce que les arêtes inférieures 8a et 10a viennent en appui contre les crêtes iliaques.

Dans cette position, le plan de centrage de la plaque 4 est confondu avec le plan sagittal du patient. Du fait de la présence des sangles de rappel élastiques, le plan de centrage des demi-ceintures 16 et 18 est également confondu avec le plan sagittal du patient.

Lors d'une seconde étape illustrée sur la figure 3b, le patient ferme la ceinture 1. A cet effet, le patient plaque les demi-ceintures 16 et 18 contre la zone lombo abdominale de sorte que :
- les extrémités proximales 16a, et 18a viennent en appui contre la zone dorso lombaire du patient, et
- les extrémités distales 16b, et 18b viennent en appui l'une sur l'autre contre l'abdomen du patient.

Dans ces conditions, les rubans textiles 22 et 24, respectivement à boucles et à crochets, ferment la ceinture 1.

Enfin, lors d'une troisième étape illustrée sur les figures 3c et 3d, le patient ajuste la pression exercée par la ceinture 1 au niveau de la zone dorso lombaire par le biais du dispositif 40.

A cet effet, le patient saisit les sangles 56, 58 par le biais des passants 60 et 62.

Le patient exerce alors une force de traction sur les sangles 56, 58 en rapprochant les extrémités libres des sangles 56, 58 vers son abdomen. En réponse à l'exercice de cette traction, les boucles 51, 52 tendent à s'éloigner l'une de l'autre.

Dans ces conditions, les brins de la sangle 54 situés entre la boucle 51 et les boucles 44, 46 s'allongent. De même, les brins de la sangle 54 situées entre la boucle 52 et les boucles 48, 50 s'allongent.

En réponse, les brins de la sangle 54 situés entre les boucles 44, et 48, et entre les boucles 46 et 50 se raccourcissent. Ainsi, à mesure que le patient exerce la force de traction sur les sangles 56 et 58, les extrémités proximales 16a et 18a se rapprochent l'une de l'autre.

Le dispositif 40 permet ainsi de transformer une force de traction exercée par le patient en une force de pression exercée par la ceinture 1 sur la zone dorso abdominale du patient.

Le patient exerce la force de traction sur les sangles jusqu'à atteindre une force de pression exercée par la ceinture 1 sur la zone dorso lombaire désirée. Une fois cette force de pression atteinte, le patient superpose les sangles 56, et 58 avec les prolongements latéraux 8, et 10 de la plaque 4 contre sa zone lombo abdominale.

Enfin, le patient fixe les sangles 56, 58 sur les demi-ceintures 16, et 18 par le biais des moyens 64. Dans ces conditions, les sangles 56, 58 déforment la plaque 4. En particulier, les sangles 56, 58 creusent les parties dorsales et latérales de la plaque 4, donnant à cette plaque 4 une forme en sablier. La pression circulaire exercée par les sangles de serrage 56, et 58 sur la taille du patient est transformée en une force de poussée sur les crêtes iliaques et la cage thoracique. Cette action mécanique prend en charge une partie du poids du thorax, atténuant ainsi la charge les vertèbres basses (L5, S1).

La forme évasée des demi-ceintures 16 et 18 est avantageuse dans la mesure où elle permet aux demi-ceintures 16 et 18 de ne pas couvrir les côtes flottantes du patient. Par « côtes flottantes » on désigne les côtes K11 et K12 du patient. Ainsi, le confort du patient est maximisé.

Par ailleurs, les bords inférieurs des demi-ceintures 16 et 18 sont largement échancrés au dessus de la cuisse (zone de l'arcade crurale) et permettent au patient une position assise confortable sans compression de la zone épigastrique du patient. Ainsi, les demi-ceintures 16 et 18 évitent l'exercice d'une compression stomacale et d'une contrainte sur le diaphragme du patient.

Les passants 60, 62 facilitent la préhension des sangles 56, 58 et l'exercice d'une force de traction sur ces sangles 56, 58 par un patient dans une direction parallèle à l'axe X.

Le mode d'action de la ceinture selon l'invention et de la plaque de maintien dorso lombaire est le suivant.

La compression de la zone lombo- abdominale située entre les crêtes iliaques et les cotes flottantes se fait par le cumul des éléments de compression suivants :
- la plaque dorsale 4 ;
- la ceinture extensible composée de deux demi-ceintures 16, 18 ;
- deux sangles inextensibles de réglage en pression 56, 58 qui passent sur les prolongements latéraux 8, 10 de la plaque dorsale 4.

Lors de la mise en tension des sangles 56, 58, les deux demi-ceintures 16, 18 se resserrent dans le dos grâce au dispositif 40, la distance - en X - les séparant diminuant. Ce dispositif 40 permet une démultiplication par trois de la force exercée par le patient, du fait des boucles 44, 51, 46 ou 48, 52, 50 offrant trois points de démultiplication. La plaque dorsale 4 coulisse dans les passants 20, évitant ainsi les plis inconfortables.

La pression exercée par la ceinture est essentiellement dorsale, dans un premier temps, avant que les deux demi-ceintures ne soient attachées l'une à l'autre sur la zone abdominale du patient.

Simultanément, les sangles 56, 58 appuient sur les prolongements latéraux 8, 10, occasionnant une déformation de la plaque dorsale 4. Celle-ci prend une forme en tablier ou en selle de cheval, présentant une double courbure lui permettant d'épouser à la fois la lordose du patient et entourant celui-ci au niveau du dos et des côtés. La ceinture s'adapte ainsi à la morphologie de chaque patient. Lorsque la ceinture est serrée, la largeur de l'ouverture 12 - dans la direction X - est diminuée. Un serrage très important peut même conduire au fait que l'ouverture 12 n'est plus visible, ses deux bords sensiblement verticaux s'étend rapprochés jusqu'à être sensiblement jointifs. Le serrage en traction de la ceinture permet de serrer la taille de la plaque dorsale 4 et du patient, sans toutefois gêner les apophyses épineuses de la colonne.

La forme géométrique en sablier, appliquée sur le segment lombo-abdominal, transforme la pression circulaire en poussée sur les crêtes iliaques et sur la cage thoracique. Cette action mécanique prend en charge une partie du poids du thorax, atténuant ainsi la charge sur les segment bas : L5/S1 et L4/L5

La plaque dorsale 4 coulissante apporte à la fois une liaison et une cohésion entre les deux demi-ceintures 16, 18 et permet d'éviter l'inconfort qui serait lié au plissement de ces demi-ceintures sur la peau ou les vêtements.

Lors du relâchement de la traction sur les deux demi-ceintures, les moyens de rappel élastique raccordant la plaque dorsale et l'une des demi-ceintures se détendent de façon symétrique, de façon que le centrage de la plaque dorsale 4 soit conservé.

Enfin, la ceinture peut comprendre des premier et second moyens de rappel élastiques d'une part et la, respectivement, première ou seconde demi-ceinture d'autre part, pour maintenir en permanence la plaque et les demi-ceintures centrées l'une avec l'autre. Des moyens sont cousus sur la plaque dorsale et sont fixés sur la demi-ceinture correspondante par des morceaux de tissu auto agrippant.

De nombreux autres modes de réalisation sont possibles.

En particulier, il est possible d'équiper la ceinture de soutien lombaire de l'invention d'une paire de bretelles. Par exemple, les bretelles comprennent une extrémité fixée sur une partie supérieure de la plaque dorsale et une extrémité libre. De préférence, les bretelles se croisent un première fois dans le dos du patient, passent sur des épaules respectives, passent sous les aisselles directement sous ces épaules, se croisent une seconde fois dans le dos du patient et viennent se fixer au niveau de l'abdomen. De telles bretelles permettent de redresser les épaules du patient, et sont particulièrement adaptées aux patients souffrant de cyphose.

Le nombre de boucles fixées aux extrémités proximales des demi-ceintures peut être différent de deux.

Les boucles peuvent être remplacées par des oeillets ou tout élément analogue.

## Revendications

1. Ceinture de soutien lombaire, comportant :
- une plaque dorsale (4) destinée à venir en appui contre la zone dorso lombaire d'un patient ;
- des première et seconde demi-ceintures (16, 18) destinées à venir en appui contre la zone lombo abdominale du patient, ces première et seconde demi-ceintures étant montées coulissantes avec la plaque (4) ;
- un dispositif (40) pour ajuster une pression exercée par la ceinture (1) sur la zone dorso lombaire du patient, ce dispositif comprenant :
- des premières boucles (44, 46, 48, 50) fixées sur les extrémités proximales des première et seconde demi-ceintures (16, 18) ;
- des secondes boucles (51, 52) libres par rapport aux extrémités proximales des première et seconde demi-ceintures ;
- une sangle dorsale (54) reçue à travers ces boucles (44, 46, 48, 50, 51, 52) en formant un maillage en ziz-zag ;
**caractérisé en ce que** :
- la plaque dorsale (4) comporte des prolongements latéraux (8, 10) comprenant une arête inférieure (8a, 10a) conformée pour venir en appui contre les crêtes iliaques du patient, et
- le dispositif (40) comprend également des première et seconde sangles de serrage (56, 58) fixées d'une part, sur une extrémité distale (16b, 18b) d'une demi ceinture (16, 18) respective, et reçues d'autre part à travers une seconde boucle (52, 51) libre respective, les première et seconde sangles de serrage (56, 58) étant destinées à être superposées aux prolongements latéraux (8, 10) de la plaque (4) contre la zone lombo abdominale du patient.

2. Ceinture de soutien lombaire selon la revendication 1, dans laquelle la plaque (4) comporte une ouverture centrale (12) pour dégager les apophyses postérieures du patient, cette ouverture centrale (12) s'étendant majoritairement selon un axe destiné à être positionné dans le plan sagittal du patient.

3. Ceinture de soutien lombaire selon l'une quelconque des revendications précédentes, dans laquelle la plaque dorsale (4) comprend un rainage (14) s'étendant selon un axe destiné à être positionné dans le plan sagittal du patient.

4. Ceinture de soutien lombaire selon l'une quelconque des revendications précédentes, dans laquelle des extrémités libres des sangles de serrage (56, 58) forment un passant (60, 62) pour recevoir un doigt du patient, de manière à faciliter l'exercice d'une force de traction sur les sangles de serrage (56, 58) par le patient.

5. Ceinture de soutien lombaire selon l'une quelconque des revendications précédentes, dans laquelle les extrémités proximales (16a, 18a) des demi-ceintures comportent chacune au moins un passant (20) à l'intérieur duquel est reçu un prolongement latéral (8, 10) de la plaque (4), de manière à permettre le coulissement des demi-ceintures (16, 18) avec la plaque (4).

6. Ceinture de soutien lombaire selon l'une quelconque des revendications précédentes, dans laquelle la ceinture (1) comprend des premier et second moyens de rappel élastiques raccordant la plaque (4) d'une part et la, respectivement, première ou seconde demi-ceinture (16, 18) d'autre part, pour maintenir en permanence la plaque (4) et les demi-ceintures (16, 18) centrées l'une avec l'autre.

## Patentansprüche

1. Lendenstützgürtel, der Folgendes umfasst:
- eine Rückenplatte (4), die dazu vorgesehen ist, gegen den Lenden-Rückenbereich eines Patienten zum Aufliegen zu kommen;
- einen ersten und einen zweiten Halbgürtel (16, 18), die dazu vorgesehen sind, gegen den Lenden-Abdomenbereich des Patienten zum Aufliegen zu kommen, wobei dieser erste und dieser zweite Halbgürtel verschiebbar mit der Platte (4) montiert sind;
- eine Vorrichtung (40) zum Justieren eines von dem Gürtel (1) auf den Lenden-Rückenbereich des Patienten ausgeübten Drucks, wobei diese Vorrichtung Folgendes umfasst:
- erste Schnallen (44, 46, 48, 50), die an den proximalen Enden des ersten und des zweiten Halbgürtels (16, 18) befestigt sind;
- zweite Schnallen (51, 52), die in Bezug auf die proximalen Enden des ersten und des zweiten Halbgürtels frei sind;
- ein Rückengurt (54), der durch diese Schnallen (44, 46, 48, 50, 51, 52) aufgenommen wird, wobei ein Zickzack-Netz gebildet wird;
**dadurch gekennzeichnet, dass**:
- die Rückenplatte (4) seitliche Verlängerungen (8, 10) umfasst, die einen unteren Steg (8a, 10a) aufweisen, der dazu angepasst ist, gegen die Darmbeinkämme des Patienten zum Aufliegen zu kommen, und
- die Vorrichtung (40) außerdem einen ersten und einen zweiten Spanngurt (56, 58) aufweist, die einerseits jeweils an einem distalen Ende (16b, 18b) eines Halbgürtels (16, 18) befestigt sind und andererseits jeweils durch eine freie zweite Schnalle (52, 51) aufgenommen werden, wobei der erste und der zweite Spanngurt (56, 58) dazu vorgesehen sind, den seitlichen Verlängerungen (8, 10) der Platte (4) gegen den Lenden-Abdomenbereich des Patienten überlagert zu werden.

2. Lendenstützgürtel nach Anspruch 1, wobei die Platte (4) eine zentrale Öffnung (12) zum Freilegen der posterioren Fortsätze des Patienten umfasst, wobei diese zentrale Öffnung (12) sich überwiegend entlang einer Achse erstreckt und die dazu vorgesehen ist, in der Sagittalebene des Patienten positioniert zu werden.

3. Lendenstützgürtel nach einem der vorhergehenden Ansprüche, wobei die Rückenplatte (4) eine Falz (14) aufweist, die sich entlang einer Achse erstreckt und die dazu vorgesehen ist, in der Sagittalebene des Patienten positioniert zu werden.

4. Lendenstützgürtel nach einem der vorhergehenden Ansprüche, wobei die freien Enden der Spanngurte (56, 58) eine Schlaufe (60, 62) bilden, um einen Finger des Patienten derart aufzunehmen, dass das Ausüben einer Zugkraft auf die Spanngurte (56, 58) durch den Patienten erleichtert wird.

5. Lendenstützgürtel nach einem der vorhergehenden Ansprüche, wobei die proximalen Enden (16a, 18a) der Halbgürtel jeweils mindestens eine Schlaufe (20) umfassen, in deren Inneren eine seitliche Verlängerung (8, 10) der Platte (4) derart aufgenommen wird, dass das Verschieben der Halbgürtel (16, 18) mit der Platte (4) ermöglicht wird.

6. Lendenstützgürtel nach einem der vorhergehenden Ansprüche, wobei der Gürtel (1) erste und zweite elastische Rückstellmittel aufweist, die die Platte (4) einerseits und den ersten bzw. den zweiten Halbgürtel (16, 18) andererseits verbinden, um die Platte (4) und die Halbgürtel (16, 18) zueinander ständig zentriert zu halten.

## Claims

1. A lumbar support belt, including:
- a dorsal plate (4) designed to bear against the patient's dorsal-lumbar area;
- first and second half-belts (16, 18) designed to bear against the patient's lumbar-abdominal area, said first and second half-belts being slidingly mounted with the plate (4);
- a device (40) for adjusting a pressure exerted by the belt (1) on the patient's dorsal-lumbar area, said device comprising:
- first buckles (44, 46, 48, 50) fastened on the proximal ends of the first and second half-belts (16, 18);
- second buckles (51, 52) that are free with respect to the proximal ends of the first and second half-belts;
- a dorsal strap (54) received through said buckles (44, 46, 48, 50, 51, 52) forming a zigzag mesh,
**characterized in that**:
- the dorsal plate (4) includes lateral extensions (8, 10) comprising a lower edge (8a, 10a) configured to bear against the patient's iliac crests, and
- the device (40) also comprises first and second tightening straps (56, 58) fastened on the one hand on a distal end (16b, 18b) of one respective half-belt (16, 18), and on the other hand received through a second respective free buckle (52, 51), the first and second tightening straps (56, 58) being designed to be superimposed on the lateral extensions (8, 10) of the plate (4) against the patient's lumbar-abdominal area.

2. The lumbar support belt according to claim 1, wherein the plate (4) includes a central opening (12) for freeing the patient's posterior processes, the majority of said central opening (12) extending along an axis designed to be positioned in the sagittal plane of the patient.

3. The lumbar support belt according to any one of the preceding claims, wherein the dorsal plate (4) comprises a scoring (14) extending along an axis designed to be positioned in the sagittal plane of the patient.

4. The lumbar support belt according to any one of the preceding claims, wherein the free ends of the tightening straps (56, 58) form a loop (60, 62) to receive one of the patient's fingers, so as to facilitate the exertion of a pulling force on the tightening straps (56, 58) by the patient.

5. The lumbar support belt according to any one of the preceding claims, wherein the proximal ends (16a, 18a) of the half-belts each include at least one loop (20) inside which a lateral extension (8, 10) of the dorsal plate (4) is received, so as to allow sliding of the half-belts (16, 18) with the dorsal plate (4).

6. The lumbar support belt according to any one of the preceding claims, wherein the belts (1) comprise first and second elastic return means connecting the dorsal plate (4) on the one hand and the first or second half-belt (16, 18), respectively, on the other hand, so as to continuously keep the plate (4) and the half-belts (16, 18) centered with respect to one another.
